(19) Europäisches Patentamt

European Patent Office

Office européen des brevets

(11) Publication number: **0 538 911 A2**

## EUROPEAN PATENT APPLICATION

(21) Application number: **92121971.3**

(22) Date of filing: **03.06.88**

(51) Int. Cl.5: **A61K 31/40**, A61K 31/195, A61K 31/66

---

This application was filed on 24 - 12 - 1992 as a divisional application to the application mentioned under INID code 60.

(30) Priority: **04.06.87 US 58140**
**26.05.88 US 198850**

(43) Date of publication of application:
**28.04.93 Bulletin 93/17**

(60) Publication number of the earlier application in accordance with Art.76 EPC: **0 316 438**

(84) Designated Contracting States:
**AT BE CH DE FR GB IT LI LU NL SE**

(71) Applicant: **MASSACHUSETTS INSTITUTE OF TECHNOLOGY**
**77 Massachusetts Avenue**
**Cambridge, MA 02139(US)**

(72) Inventor: **The designation of the inventor has not yet been filed**

(74) Representative: **Glawe, Delfs, Moll & Partner**
**Patentanwälte**
**Liebherrstrasse 20**
**W-8000 München 26 (DE)**

---

(54) **Chemical prevention or reversal of cataract by phase separation inhibitors.**

(57) A reagent comprising:

a compound having two actions, the first action decreasing phase separation temperature of lens cytoplasmic proteins below the patient's body temperature and the second action inhibiting the formation of high molecular weight aggregates,

wherein said compound is able to diffuse into the lens and is non-toxic to the lens and surrounding tissue, having no substantial effect on eye color and viscoelastic properties of the lens affecting visual acuity, and wherein preferred compounds are selected from the group consisting of succinimide, N-hydroxy succinimide, ethosuximide and pantethenic acid derivatives may be used in the in vivo prevention or reversal of phase separation cataract formation in a human or animal patient.

EP 0 538 911 A2

Rank Xerox (UK) Business Services
(3.10/3.5x/3.0.1)

Background of the Invention

This invention is generally in the area of pharmaceuticals and particularly in the area of non-surgical treatments for cataracts.

Cataract is the general term for any pathological condition in which the normal transparency of the ocular lens is substantially diminished. There appears to be a multitude of distinct aetiologies for human cataracts, involving genetic, metabolic and/or environmental factors. In most cases, the medical understanding of the physiological mechanisms which lead to cataract is incomplete.

In contrast, the cellular structure of the cell is fairly well characterized. The lens exhibits a high degree of regularity, consisting of fiber cells with hexagonal cross sections packed together to create a nearly regular parallel array of fiber cells which stretch from anterior to posterior pole. The lens fiber cells lose almost of all their intracellular organelles during the process of terminal differentiation.

Approximately 35% to 60% of the total mass of the lens consists of structural proteins, the remainder being water. More than 90% of the total lens protein consists of alpha, beta and gamma crystallins, a group of tissue-specific structural proteins found at extremely high concentrations, in excess of 300 mg/ml, in the lens cell cytoplasm. The crystallins are distributed throughout the lens along a continuous radial concentration gradient in which the concentration is greatest at the nucleus and decreases toward the lens cortex. The crystallin distribution determines the mean index of refraction and index gradient, which are in turn responsible for the optical focusing power of the lens as a whole.

Incident light is scattered in all directions by each of the individual macromolecular constituents of the lens. If the individual wavelets of the scattered light interfere destructively with one another, the lens is transparent. Destructive interference takes place in the normal lens because of the existence of short range order in the relative positions of the crystallins. If the uniformity of the protein concentration is sufficiently perturbed, a substantial fraction of the incident light is scattered in directions away from the forward direction. The scattering results in a distortion of the wave front of the transmitted light, and in turbidity of the lenticular medium, producing the observed phenomena of cataract diseases.

There are two known mechanisms on a molecular level which have been shown to be responsible for the loss of short range order of the relative position of lens proteins. The first mechanism has to do with the formation of high molecular weight aggregates. The first quantitative investigation of this mechanism was made in 1971 by G. B. Benedek in Applied Optics 10, 459 (1971).

In 1973, Jedziniak et.al. reported in Exp. Eye Res. 15, 185-192 (1973) the presence of high molecular weight protein aggregates in the lens and measured the molecular weight of these aggregates. They found the molecular weight to be greater than approximately $150 \times 10^6$ grams per mole. The aggregates were present in the soluble fraction of aging normal and cataractous human lens. This protein population represents approximately 5% of the total soluble protein in lenses up to age 75, increasing by a factor of two to three in lens at an age greater than 75 years and in cataractous lens. Differences in the amino acid composition of the heavy molecular weight soluble aggregates and the calcium ion concentration were found when the normal and cataractous lens were compared.

The second known mechanism for formation of cataracts is the phenomena of protein/water separation first enunciated by Tanaka and Benedek in Invest. Opthal. 14(6), 449-456 (1975). Following the discovery of phase separation cataract, Benedek et.al reported in Phil.Trans.R.Soc. Lond.A 293, 329-340 (1979), groups of reagents which can induce opacification at body temperature and which can reverse opacification due to light scattering. Their work was based on the reversal or creation of "cold cataract" in calf lens, a phenomena described by Zigman and Lerman in Nature, London 20, 662-663 (1964). The work of Tanaka and Benedek demonstrated that the phenomena of "cold cataract" was in fact the result of the separation of the lens proteins into coexisting phases. As the temperature of isolated calf lens is lowered to about 17°C, the turbidity of the nuclear region increases rapidly until multiple scattering occurs and the lens becomes opaque. The opacification is reversed when the temperature is raised above the temperature "$T_{cat}$" at which phase separation occurs. The reagents reported to increase $T_{cat}$ include the monovalent salts LiCl, NaCl, KCL and CsCl, heavy water $D_2O$, and methanol. By selectively choosing the concentration of these opacifying agents, the cataract temperature of the calf lens can be raised close to or above body temperature. The reagents that decrease $T_{cat}$ include glycerol, ethylene glycol, 1,3-butanediol, 1,4-butanediol, urea, guanidine hydrochloride and glycine. However, while these reagents are useful for conducting laboratory experiments, they are not useful on a clinical basis due to side effects and/or their transient effect on the phase separation temperature.

It was proposed by Clark and Benedek in Invest. Opthal.Vis.Sci. 19(7), 771-776 (1980) that reversible clarifying reagents such as glycols non-covalently interact with lens constituents so that phase separation in the lens cell cytoplasm occurs at a reduced temperature. Aldehydes and crosslinked acrylamide were reported to irreversibly interact with the lens constituents to reduce the phase separation temperature. However, both aldehydes and uncrosslinked acrylamides are toxic. Aldehydes also cause a change in the lens color and an increase in hardness and response to light, effects which are related to a crosslinking process. As a consequence, they do not meet FDA requirements. The use of these reagents to permanently modify lens structure is the subject of U.S. Patent Nos. 4,351,826, 4,474,817, and 4,526,789.

Recently Vitamin E has been tested in vitro as a reagent for altering lens opacities. It may act through an effect on the physical properties of the cell membranes. Unfortunately, even if the effect is reproducible in vivo, Vitamin E has limited use since it is toxic in high concentrations. These results were reported by Ross et al in Exp.Eye Res. 36,645-653 (1983); Creighton et al in Exp.Eye Res. 40, 213-222 (1985); and Libondi et al in Exp.Eye Res. 40,661-666 (1985).

None of the compounds described above were tested in vivo. A compound which was tested in vivo was reported by Menard et al., in Int. J. Radiation Oncology Biol.Phys. 12,1483-1486(1986). This preliminary work used systemic injection of a radioprotective drug prior to exposure to x-irradiation to prevent subsequent radiation cataract formation. The criteria examined by Menard et al. were opacity, formation of high molecular weight aggregates, and loss of soluble protein. Although the drug did successfully protect against radiation cataracts, the authors note that the mechanism by which the compound worked was not apparent from the experiments and that there was no indication if the drug would work with any other kind of cataract, or if administered following initiation of cataract formation, or if administered by a different mode.

Cataracts are the second leading cause of blindness worldwide. Despite the theories which have been proposed and the results demonstrated using reagents such as glycols and acrylamide, or even the in vivo work with the radioprotective drug, surgery remains the primary form of treatment. For example, in recent years over 1,000,000 operations for the removal of cataracts have been performed annually in the United States. To date, no one has developed a compound for topical or oral administration which has been demonstrated to prevent cataracts of diverse origin in vivo. Further, no one has ever successfully demonstrated in vivo reversal of the initiation of cataract formation by administration of a reagent administered by any mode.

## Summary of the Invention

The present invention relates to an in vivo method and reagents, for decreasing the phase separation temperature and inhibiting the formation of high molecular weight aggregates in eye lenses to thereby prevent or reverse phase separation cataract formation. These reagents meet the following criteria: they are able to diffuse from an aqueous solution into the lens and exert an effect over a reasonable period of time; they are not toxic to the lens or surrounding tissue when delivered systemically or topically to the eye; and they do not change the eye color or viscoelastic properties of the lens in a manner detrimental to visual acuity. In the preferred form, the reagents are applied locally (i.e., topically) or systemically (i.e., orally or by injection) to minimize the effective dose and side effects.

The specific effect of the reagents is to inhibit spatial fluctuations in the index of refraction by stabilizing the short range order in the lens proteins. This is accomplished by a decrease in the phase separation temperature and by a suppression of the formation of high molecular weight aggregates. Temperatures for which phase separation occurs can be determined from a coexistence curve distinguishing the homogeneous from the heterogeneous phases. At temperatures outside (above) the coexistence curve, the lens cytoplasm exists as a homogeneous, transparent phase. At temperatures within (below) the coexistence curve, the cytoplasm separates into regions which are rich and poor in the constituent proteins. These regions, which have different indices of refraction, scatter light strongly and produce opacification. A technique for constructing phase diagrams for isolated lens cytoplasmic homogenate is reported by J.I. Clark and G.B. Benedek in Biochem. Biophys. Res. Com, 95(1), 482-489 (1980). A method for measuring the phase separation temperature of intact lenses is described by Clark et al. in Invest. Ophthal. Vis. Sci. 22(2), 186-190 (1984).

Pathologic cataracts are characterized by disruption of membrane structure, the formation of high molecular-weight aggregates, and functional deterioration. These changes occur well after the first changes in phase separation temperature occurs. The mechanism relating the two, if any, is not clear. However, the phase separative temperature serves as a useful indicator of the earliest stages of cataract formation. The presence of high molecular weight aggregates, which scatter light and cause opacification, can be determined from the measurement of the intensity autocorrelation function of laser light scattered from the lens, where the width of the scattered light spectrum or the reciprocal of the correlation time of the scattered light intensity fluctuation decreases with the concentration of high molecular weight aggregates.

One technique for determining the presence of these aggregates is described by M. Delaye, J.I. Clark and G.B. Benedek in "Identification of the Scattering Elements Responsible for Lens Opacification in Cold Cataracts" in Biophys. J. 37, 647-656 (1982).

Examples of reagents according to the present invention which have been demonstrated to meet the desired criteria are succinimide, N-hydroxy succinimide (NHS), ethosuximide, pantethenic acid derivatives such as pantethine, WR-77913 S-3-(amino-2-hydroxypropyl) phosphorothioate, or other related phosphorothioate compounds such as WR-2721, (S-2-(3-aminopropylamino) ethyl phosphorothioate, applied topically or systemically by injection or oral administration.

Brief Description of the Drawings

Figure 1 is a photograph of control rat eyes compared with cataractous rat eyes, rat eyes treated with WR-77913 and rat eyes treated with pantethine, after exposure to x-irradiation.

Figure 2 is a graph of the transmittance of a cytoplasmic homogenate as a function of temperature ($^{o}$C) for samples containing 0.0 mM, 10 mM, 25 mM and 50 mM galactose.

Figure 3 is a graph of the change in phase separation temperature ($^{o}$C), $T_c$, versus concentration (0 to 50 mM) of galactose, WR-77913 and WR-2721.

Figure 4 is a graph of the change in phase separation temperature ($^{o}$C), $T_c$, of a calf lens versus concentration of NHS (mM).

Figure 5 is a graph of the change in phase separation temperature ($^{o}$C), $T_c$, versus concentration of naturally occurring phase separation inhibitor (arbitrary units).

Figures 6(a) and 6(b) are graphs of the change in phase separation temperature ($^{\circ}$C), $T_c$, versus concentration of succinimide and ethosuximide, respectively, for concentrated lens homogenates.

Figure 7 is a graph of the change in phase separation temperature ($^{\circ}$C), $T_c$, versus concentration of succinimide applied to freshly removed whole rat eyes.

Figure 8 is a graph of lens transmittance versus temperature ($^{\circ}$C) determined following in vivo topical applications of succinimide.

Figure 9 is a graph of the change in phase separation temperature ($^{\circ}$C), $T_c$, versus concentration of pantethine in vitro.

Detailed Description of the Invention

The present invention is the discovery that reagents which decrease phase separation temperature can prevent or reverse cataract formation in vivo, regardless of the source of the cataract. The phase separation temperature is defined as the temperature at which, at a given protein concentration, the cytoplasm will segregate into coexisting phases. The temperature is determined from the coexistence curve in a phase diagram plotting temperature ($^{\circ}$C) against protein concentration, where segregation occurs at a temperature and concentration under the curve. Prior to this time, phase separation temperature was suggested experimentally as a sensitive indicator of cataract formation.

This invention pertains to a variety of reagents which, when tested in vivo, have been found to prevent formation of opacities, high molecular weight aggregates and other physical characteristics of cataracts, if administered topically or systematically by injection prior to exposure of the lens to radioactivity. Reagents which alter phase separation temperature in vitro have also been demonstrated. Prior to the present invention, reagents which act directly in vivo to decrease the phase separation temperature of proteins in the lens cytoplasm, thereby preventing or reversing phase separation cataract formation, have not been described.

There are several established animal models for human cataracts, including cataracts caused by x-irradiation, a 50% galactose diet (galactosemic cataracts), and cataracts in the diabetic rabbit or rat.

X-irradiation has been found to increase the phase separation temperature prior to causing the formation of high molecular weights aggregates, serious morphologic damage, and changes in lens permeability and lens transport. These changes are characteristic of many pathologic cataracts.

The method, described in detail by Clark et al. in Invest. Ophthalmol. 21, 186 (1982) for inducing radiation cataracts is as follows: X-ray cataracts are produced in one eye of a New Zealand white rabbit by irradiating the eye with a single 2000 rad (85 kVp, 5 mAmp) dose when the animal is 5 to 6 weeks of age. Under these conditions a mature cataract develops in the irradiated eye 8 to 9 weeks after irradiation. The unirradiated contralateral lens has been found to receive less than 50 rad of irradiation and is used as a control. At measured intervals after irradiation, the irradiated and unirradiated lenses are removed from the rabbit eyes. The lenses are placed immediately in silicone oil (Dow Corning 550) and kept at approximately 5°C.

The phase transition temperatures, $T_c$, of the irradiated and the unirradiated lenses are determined by means of laser transmittance. The pair of lenses are placed in a cuvette filled with silicone oil, which is then mounted on a movable temperature-controlled stage that is directly in the path of a laser beam, with the anterior surface facing the beam. The transmittance can be measured with this apparatus as a function of temperature at every region in the opaque lens. When the transmittance in the region of densest opacity reaches 75% of its maximum value, this temperature is defined as the $T_c$ of the lens. By plotting the $T_c$ of the irradiated lenses and that of the unirradiated control lenses at different times after irradiation, one can compare the difference between the $T_c$ of the normal and irradiated lens during cataract development. Other methods of determining the phase separation temperature include light scattering determinations.

A cytoplasmic phase separation is associated with the earliest stages of cataract formation produced by X-irradiation. The phase separation occurs over a narrow temperature range and is characterized by the phase separation temperature, $T_c$. In normal lenses, the $T_c$ is well below body temperature. In X-irradiated lenses, $T_c$ increases during the early stages of cataract formation. As the cataract progresses, membrane function is disrupted, ion levels change, high molecular weight aggregates are formed and an advanced, irreversible cataract forms.

Cataract produced by irradiation is not the only cataract that is associated with increasing $T_c$. Cataract in the Philly mouse (Clark and Carper, Proc.Natl.Acad.Sci.USA 84,122-125 (1987)), and cataract produced by galactosemia (Ishimoto et al., Proc.Natl.Acad.Sci.USA 76,4414-4416(1979)), hypoglycemia (Tanaka, Invest.Ophthalmol.Vis.Sci. 24,522-525 (1983)), and cyanate (Crompton et al., Exp.Eye Res. 40,297-231 (1985)) are all associated with an increase in Tc.

The chemical reagents succinimide (chemical formula $C_4H_5NO_2$) and ethosuximide (chemical formula $C_7H_{11}NO_2$) have been found to strongly reduce the phase separation temperatures of solutions comprising concentrated bovine lens nuclear homogenate having a concentration of about 280 mg/ml. Succinimide has also been shown to reduce the phase separation temperature of lenses removed from whole rat eyes following incubation of the freshly extracted eyes in succinimide-containing solutions.

Additionally, succinimide has been found to cause reversal of cataracts produced in vivo by sodium selenite. This is the first time that in vivo cataracts have been reversed via application of phase separation inhibitors.

In addition to succinimide, pantethine has been demonstrated to be a phase separation inhibitor that prevents cataract produced by X-irradiation in vivo. These results are especially encouraging since pantethine, and pantethenic acid from which is is derived, are approved for human use and widely available without restriction.

Protection by WR-77913 against radiation-induced cataract formation in rats was observed following intraperitoneal (i.p.) administration of drug (1160 mg/kg) 15 to 30 min before exposure to 15.3 Gy of Cs-137 whole head irradiation, as presented by T. W. Menard et al at the Chemical Modifiers for Cancer Treatment Conference, Clearwater, FL on October 20-24, 1985 and reported in Int. J. Radiation Oncology Biol. Phys. 12, 483-286 (1986). Control groups included irradiated, non-protected animals, and sham-irradiated aging controls. Protection was documented photographically and by analysis of eye lens constituents. All non-protected irradiated animals developed dense cataracts throughout the lens between 90-120 days post-irradiation, while WR-77913 protected animals developed minimal lens opacification through 200 days post-irradiation. Non-opacification in aging controls was seen.

Subsequent lens protein analysis by Lowry assay and size exclusion HPLC showed radioprotected and aging control animals were similar in protein content, distribution of total and soluble protein, and degree of lens hydration, as reported by Osgood, Menard, Clark and Krohn in Invest.Opthalmol. 27, 1780-1784 (1986). This contrasted significantly with cataractous lenses of non-protected animals. In cataractous lenses, the soluble protein concentration in the 25 to 43,000 dalton range was approximately 10% of that found in radioprotected or aging control lenses. Hydration was substantially higher in cataractous lens. No investigation of the effect of WR-77913 on phase separation temperature was reported, however, nor the effect of mode, timing or dosage of administration.

The following is a summary of the method and results obtained by systematic administration of the radioprotective compound WR-77913 prior to irradiation, showing prevention of radiation-induced cataractogenesis.

Methods and Materials:

Animals: Male Sprague-Dawley rats, 7-9 weeks old, were housed separately in an environmentally controlled room with an artificial light cycle (6 am to 6 pm). Three to five days acclimation was allowed prior to initiation of experiments. Food and water were provided ad libitum throughout the study. Irradiated animals were monitored daily for the first 10 days following initiation of experiments, and subsequently on an alternate day basis until the time of sacrifice.

Drug Preparation and Administration: Unlabeled S-3-(Amino-2-hydroxypropyl) phosphorothioate used was obtained from the Developmental Therapeutics Program, Division of Cancer Treatment, NCI. Drug purity was assessed by thin layer chromatography and melting point. 300 mg drug was dissolved/ml filter sterilized calcium-magnesium free phosphate buffered saline (0.13 M PBS, pH 7.24) immediately prior to use and administered at a dose of 1160 mg/kg body weight. In control experiments, non-protected, irradiated animals and aging control animals received an equal volume of PBS only.

Animal Irradiation: Gamma irradiation was performed with a Cs-137 teletherapy unit fitted with an 18 x 18 cm collimator. Irradiated animals received a single exposure of 15.3 Gy (0.63 Gy/min). Rats were unanesthetized and positioned in wedge-shaped lucite restraining devices so that the entire head was under the collimator. Dosimetry was measured with a 100 R ionization chamber. Sham-irradiated aging control animals were treated similarly to irradiated animals but without exposure to radiation.

Assessment of Eye Protection: The appearance of lenses from experimental groups was documented by whole eye and slit lamp photographs, 154 days post-irradiation using a photo-biomicroscope. Rats were anesthetized with pentobarbital (30 mg/kg, i.p.) and the pupils dilated with 5% phenylephrine and 0.5% tropicamide.

Protein analysis:

Rats from each group were sacrificed in ether chambers up to 210 days after receiving treatments, and the eyes enucleated and placed on ice. Lenses were dissected from the eye within 30 min and weighed. To study the content and molecular weight distribution of rat lens proteins, extracts were prepared by homogenizing individual lenses in 1 ml Wheaton homogenizers (Wheaton, Millwood, NJ) with 0.5 ml of 0.10 M sodium sulfate/0.02 M potassium phosphate elution buffer (pH 6.9). The lens homogenate was transferred to microcentrifuge vials and the homogenizers rinsed twice with 0.25 ml of elution buffer. The total volume of 1.0 ml was vortexed for 5 sec and centrifuged for 20 min at 17,000 rpm. The supernatant was decanted and filtered through 0.22 micron Millipore filters (Millipore, Bedford, MA) and saved for protein analysis and size exclusion high performance liquid chromatography (HPLC). The pellet was resuspended in 0.1 N NaOH. The amount of protein in the soluble and insoluble fractions was measured using the Lowry method standardized against bovine serum albumin.

A Perkin-Elmer Series 4 liquid chromatography module (Perkin-Elmer-Norwalk, CT), a Hewlett-Packard HP 1040A photodiode array spectrophotometer (Hewlett-Packard, Palo Alto, CA), and a TSK G3000SW, 30 cm by 7.5 mm column with a 10 cm guard column was used as the size-exclusion HPLC system. The buffer used for homogenization also served as the mobile phase with a flow rate of 0.5 ml/min. The injection volume for each sample was 10 microliters and absorbance was monitored at 280 nm. The TSK column was calibrated with known molecular weight standards.

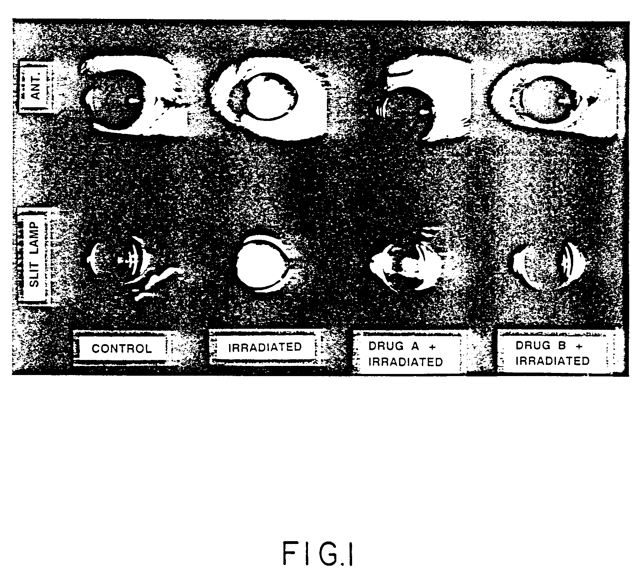

Figure 1 is a photographic representation of the protective effect that WR-77913 (Drug A) and pantethine (Drug B) has against cataract formation in x-irradiated rat lenses. Anterior and slit lamp views of rat eyes from control, irradiated, and irradiated and drug-treated animals are shown 154 days after treatment. Control rat lenses remained transparent throughout the study. Irradiated rats which received no drug treatment developed moderate lenticular opacities within 90 days of receiving gamma radiation. Lens opacification progressed to mature cataracts by 120 days post-irradiation in all animals which received no WR-77913 or pantethine. Rats protected by WR-77913 or pantethine were noted to have only very slight lenticular opacities when photographed 154 days after irradiation. The appearance of the lens in the drug-treated rats remained stable until the animals were sacrificed at 210 days post-irradiation.

Lowry protein analysis demonstrated marked differences in total protein, soluble protein, and degree of hydration for cataractous lenses compared to radioprotected and aging control animals which were similar. For purposes of comparison the lens composition for each experimental group was normalized to the aging control group and results expressed as percentages. In both radioprotected and aging control lenses, total protein comprised approximately 40% of the lens weight compared to 20% for the cataractous lenses. Of the total protein, the soluble fraction in aging control animals and radioprotected animals was 68 and 54%, respectively. In contrast, the soluble protein content in lenses of non-protected rats comprised less than 5% of the total protein. Water accounted for approximately 80% of the cataractous lens weight compared to 60% of the lens weight from aging control and radio-protected animals. The lens weight from radio-protected and non-protected rats was not significantly different.

While the radioprotected lenses consistently showed less soluble protein than aging control lenses, the HPLC elution profile demonstrated the similarity between these experimental groups which contrasted with the findings from cataractous lenses. Both radioprotected and aging control elution curves demonstrated five similar peaks between 18 and 27 min. These peaks correspond to proteins with molecular weights of approximately 158,000, 43,000, 32,000, 20,000 and 15,000 daltons. The elution profile from non-protected cataractous lenses demonstrated sharp reduction in soluble proteins. The lens protein concentration in the 25-43,000 dalton range was 10% of that found in radioprotected or aging control lenses. There was no measurable soluble protein below 25,000 daltons. The hydration and dramatic increase in the ratio of insoluble to soluble protein in the cataract is due primarily to loss of soluble protein.

This work demonstrated that the gross pathological defects caused by irradiation of the eye could be prevented by systemic injection of a radioprotective drug. At the time the work was published by Menard et al., Int. J. Radiation Oncology Bio. Phys. 12, 1483-1486 (1986) and Osgood et al., Invest. Ophthal. Vis. Sci., 27, 1780-1784 (1986), a number of mechanisms for radiation damage were proposed, including damage to cells of the germinal epithelial zone of the lens or a decrease in the lens fiber enzymatic reducing systems. In their discussion, Osgood et al. noted that WR-77913 protected the lens by stabilizing the composition of soluble proteins.

This compound, WR-77913, as well as succinimide, N-hydroxy succinimide, ethosuximide, pantethine and WR-2721, has now been found to prevent radiation cataract and to decrease the phase separation temperature of the lens cytoplasmic proteins. Additionally, WR-77913, succinimide and ethosuximide have further been demonstrated to be effective when applied topically (locally) in a suitable buffer. Also, WR-77913 has been found to be effective when administered systemically for the treatment of diabetic cataracts and galactosemic cataracts. Important elements for the successful use of the drug are the selection of the mode and dosage of administration and the timing of administration. The compound must be applied before the formation of the high molecular weight aggregates. It should be applied as soon as possible after detection of any rise in phase separation temperature. Dosage is determined by the mode of administration. Local or topical application in an acceptable physiological buffer is preferred since a lower total dosage is required. Systemic injection can produce undesirable side effects. Oral administration in an appropriate encapsulated or tablet form is also acceptable. The required amount is determined by measuring the decrease in phase separation technique ($°C$) per mole. For in vivo application, it is necessary to decrease and maintain the phase separation temperature at less than body temperature.

Example 1: Decrease in Phase Separation Temperature by WR-77913, WR-2721 and Galactose.

The following demonstrates the effect of three compounds, galactose, WR-77913 and WR-2721 on the phase separation temperature of homogenates of lens tissue.

The measurement of $dTc/dC$, the difference in phase separation temperature produced by the reagent, is useful as a method for identifying potential reagents for preventing cataract in vivo. Materials and Methods.

Preparation of Homogenates: Native lens homogenate is prepared from lenses dissected from fresh calf eyes and cooled to 4°C. At this temperature the lens nucleus opacifies and the transparent cortical layers are easily removed from the opaque nucleus. The nuclear samples are chopped into small pieces and a few drops of sodium azide (final concentration 0.04%) added to prevent bacterial contamination. The samples are then homogenized. Fifty or more lenses are used for each preparation of homogenate. This preparation has been shown to be very similar to native lens cytoplasm in physical, structural and biochemical properties. The lens homogenates are used to test the effect of chemicals on $Tc$ because the chemicals can be mixed uniformly in the cytoplasm at known concentrations. Studies using intact lenses must consider the partition coefficient in membranes, and the ability to penetrate into the cytoplasm in determining the final concentration of reagent in the cytoplasm.

Preparation of Experimental Samples: Galactose is obtained from Sigma Chemical Co. (St. Louis, MO). WR-77913 (S-3-(amino-2-hydroxypropyl) phosphorotioate) and WR-2721 (S-2-(3-aminopropylamino)ethyl phosphorotioate) are supplied by the Developmental Therapeutics Program, Division of Cancer Treatment, NCI. The reagents are dissolved in distilled water and added to the homogenate by mixing one part of the solution with 9 parts homogenate. Controls are prepared with distilled water only. In all samples the concentration of homogenate is constant. The samples are gently homogenized to mix the additives with the homogenate. Previous studies have shown that dilutions of 10% or less produce no additional background scattering and that the change in $Tc$ is caused by the addition of chemical reagents and is not simply the effect of dilution. The concentrations reported in the results are the final concentrations of the chemicals in the homogenates. The $Tc$ values are measured over a concentration range between 0 mM and 50 mM for each reagent.

Measurement of Phase Separation Temperature, Tc: The intensity of the light transmitted through the samples is measured using a simple LASER spectrometer. The relative transmittance is calculated as intensity, I, measured at any temperature, divided by the maximum intensity, $I_{max}$. Transmit tance = $I/I_{max}$. The transmittance is measured over a range of increasing temperatures from 0°C to 20°C. It is important to allow the sample to equilibrate at each temperature before recording the intensity.

The transition from Transmittance = 0.0 (opaque) to Transmittance = 1,0 (transparent) occurs over a narrow temperature range. The temperature at which Transmittance = 0.5 is arbitrarily defined as Tc. The values of Tc obtained by this method are used in the determination of dTc/dC, a measure of the effect of each chemical on Tc.

Determination of dTc/dC: The effect of the chemical on Tc is defined as the change in Tc, with change in concentration of the chemical, dTc/dC. The Tc of the control, which contains only water, and the decrease in Tc produced by the chemical additives are determined. The mean decrease in Tc and the standard deviation are determined for each concentration. A linear regression is used to determine the best linear fit of the data and the correlation coefficient. The slope of the line is defined as dTc/dC.

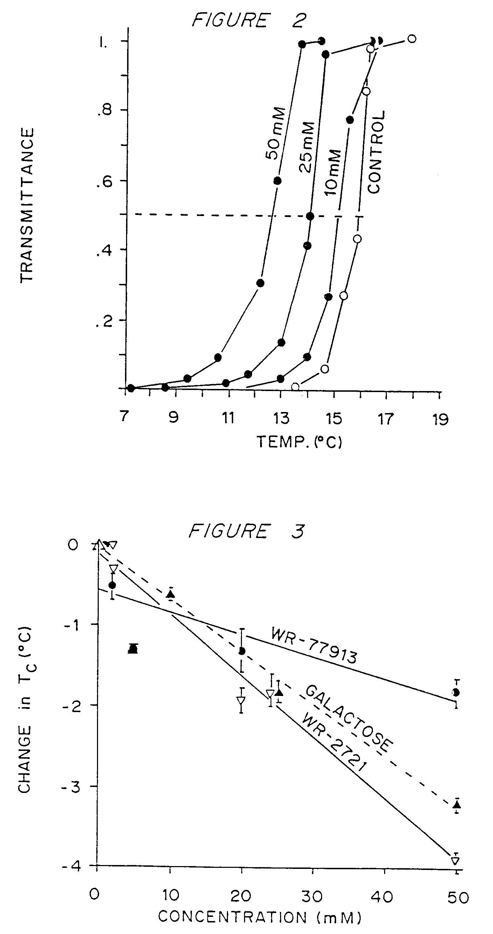

Fig. 2 is an example of a concentration series used to determine the decrease in $T_c$ at various concentrations of a compound, in this case, galactose. A diet of excess galactose results in cataract formation, presumably via a metabolic mechanism similar to that of the diabetic cataract. Unexpectedly, a high galactose diet delays formation of cataracts after irradiation. These phenomena are distinct from the results shown in Fig. 2 which demonstrate the effect of galactose itself, not the metabolic products, on the phase separation temperature of the lens. The transmittance of the cytoplasmic homogenate is plotted as a function of the temperature for samples containing 0.0 mM, 10 mM, 25 mM and 50 mM galactose. The Tc for each sample is indicated by the intersection of each plot with the dashed horizontal line at Transmittance = 0.5. The Tc was 15.6°C in the control, 15.0°C in the sample containing 10 mM galactose, 13.8°C in the sample containing 25 mM galactose and 12.4°C in the sample containing 50 mM galactose. At temperatures above 17°C all samples were completely transparent. The decrease in Tc relative to the control was -0.6°C for 10 mM, -1.8°C for 25 mM and -3.2°C for 50 mM galactose. Similar concentration series are used to measure the decrease in Tc produced by other reagents.

Fig. 3 shows the decrease in Tc produced by galactose, WR-77913 and WR-2721 over the concentration range of 0 to 50 mM. The slopes of the regression lines through the data are the dTc/dC for each compound. dTc/dC was -65°C/mole for galactose, -28°C/mole for WR-77913 and -76°C/mole for WR-2721. The correlation coefficient was 0.997 for the galactose data, 0.800 for WR-77913 and 0.993 for WR-2721.

Example 2: In Vivo Administration of WR-77913 Inhibits Streptozotocin-Induced Diabetic Cataracts.

WR-77913 was administered by i.p. injection 30 minutes prior to initiation of diabetic cataract by injection of 60 mg. streptozotocin. The WR-77913 was administered as a single injection of 1160 mg/Kg in PBS buffer. Opacities were observed in the streptozotocin-treated animals approximately 42 days after injection. No cataracts were observed in the WR-77913 treated animals.

Example 3: In Vivo Administration of WR-77913 Inhibits Galactosemic Cataracts.

WR-77913 was administered to rats that were induced to form galactosemic cataract. Every 2 days after initiation of the galactosemic diet, WR-77913 was administered by i.p. injection at a dose of 450 mg/Kg. On day 10 after starting the galactosemic diet, cataract appeared in the rats without WR-77913. No cataracts were observed in WR-77913 treated rats.

Example 4: In Vivo Administration of WR-2721 Inhibits Cataract Due to X-Irradiation.

WR-2721 is an amino-phosphorothioate similar to WR-77913, which decreases the phase separation temperature, as shown in Fig. 3. A single i.p. injection of 500 mg/Kg WR-2721 15 minutes prior to X-irradiation prevented formation of cataracts. The effect of WR-2721 is very similar to that of WR-77913 in the sense that it also prevented hydration, formation of high molecular aggregates and loss of soluble protein.

Example 5: In Vivo Administration of WR-77913 Inhibits Cataract Induced by X-Irradiation When Administered Following Irradiation.

WR-77913 was administered to X-irradiated animals 30 minutes following irradiation. The WR-77913 was administered as a single i.p. injection at a dose of 1160 mg/Kg. After 90 days, cataract appeared in the animals without WR-77913. No cataract was observed in animals with WR-77913. This result indicates that WR-77913 and other phase separation inhibitors can be successfully used to prevent cataract when administered following the initiation of opacification.

Example 6: Local (Topical) In Vivo Administration of WR-77913 Inhibits Cataract Induced by X-Irradiation.

WR-77913 was administered locally as eyedrops to the eyes of X-irradiated rats 60 minutes prior to X-irradiation. The eyedrops contained 600 mg/ml WR-77913 in PBS. The total amount of compound was about 0.5 ml or 1200 mg/kg. After 90 days X-ray cataracts appeared in the rats without WR-77913. No opacities were observed in the WR-77913-treated rats indicating that local administration of WR-77913 and other phase separation inhibitors can be effective in preventing cataract.

Example 7: Topical In Vitro Administration of NHS to Lower the Phase Separation Temperature of the Lens.

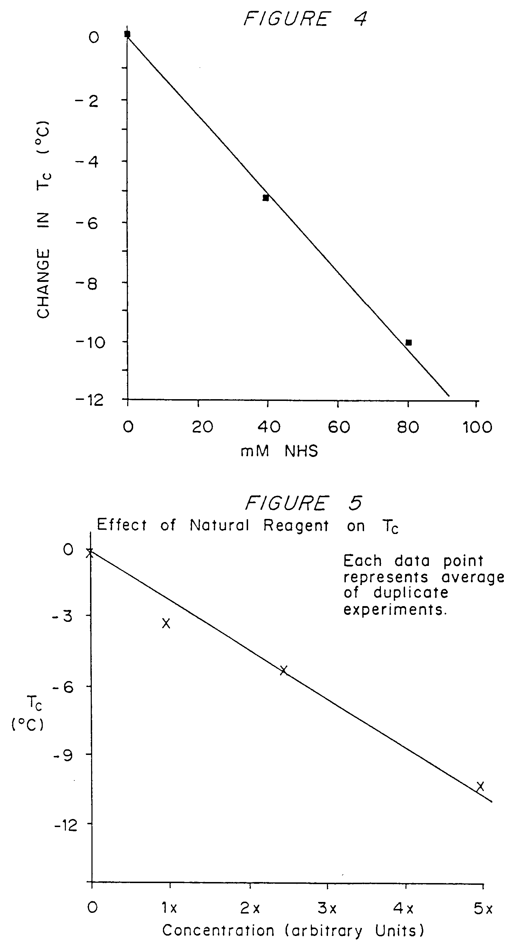

The decrease in phase separation temperature (- $T_c$) in a calf lens is plotted in Fig. 4 as ordinate versus NHS concentration in mM plotted as abscissa. Both test lens and control were placed in aqueous solutions containing 0.1 M phosphate buffer, 2% dimethyl sulfoxide (DMSO) at pH 7.0. The solution containing the test lens also contained a controlled amount of NHS ranging from 0-80 mM in concentration. After 48 hours incubation and subsequent dialysis of unbound NHS the difference (- $T_c$) between the phase separation temperature of the test lens and the control was measured as a function of NHS concentration.

Bovine lenses treated with NHS preparations for 40 hours in vitro have phase separation temperatures as much as 10°C below that of a control lens, even following extensive dialysis of the lens, a test for permanancy of Tc suppression.

Example 8: Topical In Vivo Administration of NHS to Lower the Phase Separation Temperature of the Lens.

Fifteen day old Sprague-Dawley rats were used to test N-Hydroxy Succinimide, NHS, on the decrease of Tc in vivo. Fifteen day old animals were selected because the normal Tc is above 30°C at this age. 100 mM NHS in 0.154 M phosphate buffer, pH 7.0, was applied to one eye of each rat over a 24 hour period. Phosphate buffer alone was applied to the opposite eye as a control. The solutions were applied approximately every 30 min from 4 pm to 9 pm, and again every 30 min from 7:30 am to 4 pm the next day to the eyes as drops (containing approximately 6 mg), using a syringe (a total of approximately 116 mg). At 4 pm, 24 hours after beginning the experiment, all 3 rats were sacrificed and the lenses were removed and placed in silicon oil. The Tc was measured in each lens:

| Tc (°C) | | | |
|---|---|---|---|
| | Control Eye (no NHS) | NHS | Tc |
| Rat 1 | 33.5 | 32.0 | -1.5 |
| Rat 2 | 39.0 | 36.2 | -2.8 |
| Rat 3 | 38.5 | 35.5 | -3.0 |

Fig. 4 is a graph of the decrease in Tc as a function of NHS concentration.

Example 9: A Natural Phase Separation Inhibitor for Prevention of Cataracts.

One hypothesis has been that WR-77913 mimics the action of a natural, endogenous constituent that protects against opacification. In support of this hypothesis, a naturally occuring substance extracted from normal lens tissue having a molecular weight of less than 2000 Da has been used to decrease the phase separation temperature, Tc. Figure 5 shows the strong inhibitory effect of the natural compound. The compound was added to lens homogenate as described in Example 1.

9

The substance is extracted from a lens homogenate which is centrifuged at approximately 250,000 g and the soluble supernatant filtered through a filter having a 2000 m.w. cutoff. It is important to prepare the homogenate without added buffer or salt, at physiological pH (7.0 - 7.2). The active compound is believed to be a metabolite in anaerobic glycolysis and is inactivated by a protease. On this basis, it is expected that the active compound will also be present in other natural sources including yeast and bacteria.

A natural reagent may have a variety of advantages: toxicity is less of a problem than with a synthetic phase separation inhibitor; natural reagents may provide additional insight into the action of the phase separation inhibitors, in vivo; and natural reagents provide simple model compounds that can be modified to increase anti-cataract activity.

Example 10: In Vitro Reduction of Phase Separation Temperature Using Succinimide and Ethosuximide

Concentrated bovine nuclear homogenate was assayed to be approximately 280 mg/ml. Solutions of succinimide and ethosuximide in phosphate buffer (0.1M, pH 7) were prepared having concentrations of 1.2, 0.8, 0.6, 0.4, 0.2, 0.08 and 0.04 Molar. Ten microliters of the succinimide solutions were added to a first set of 90 microliter samples of the concentrated nuclear homogenate. Ten microliters of the ethosuximide solutions were added to a second set of 90 microliter samples of the concentrated nuclear honogenate. The resulting solutions were found to be about 252 mg/ml, and the resultant succinimide and ethosuximide concentrations were about 120, 80, 60, 40, 20, 8 and 4 mM. A control sample was prepared by adding 10 microliters of buffer to 90 microliters of concentrated nuclear homogenate.

The samples were allowed to stand for about 24 hours and the $T_c$ values for each were then determined. Both the succinimide and the ethosuximide were found to decrease the $T_c$ in each of the homogenate samples. The change in $T_c$ as it related to the concentration of the reagent was found to be the same for both succinimide and ethosuximide. This relationship is given as:

$$\frac{dT_c}{d[C]} = 53.3 °C/Mole.$$

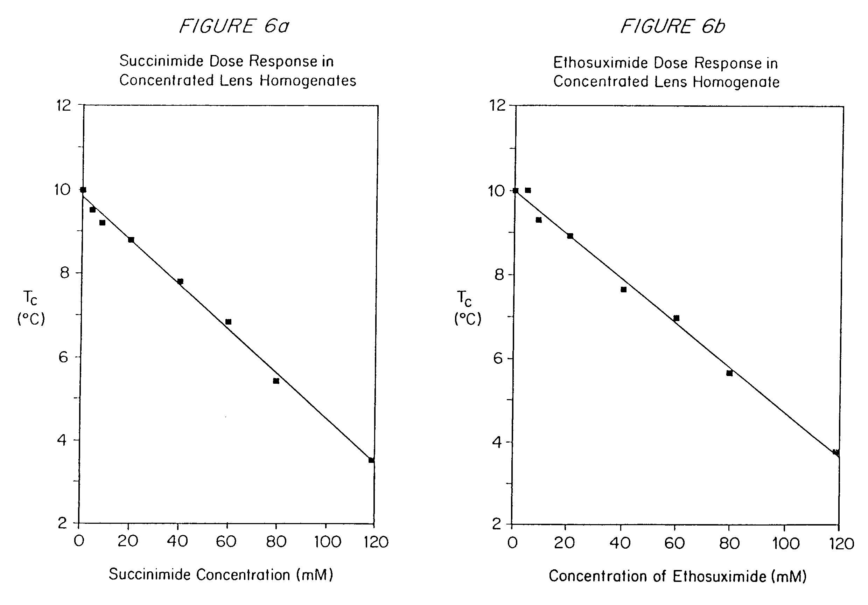

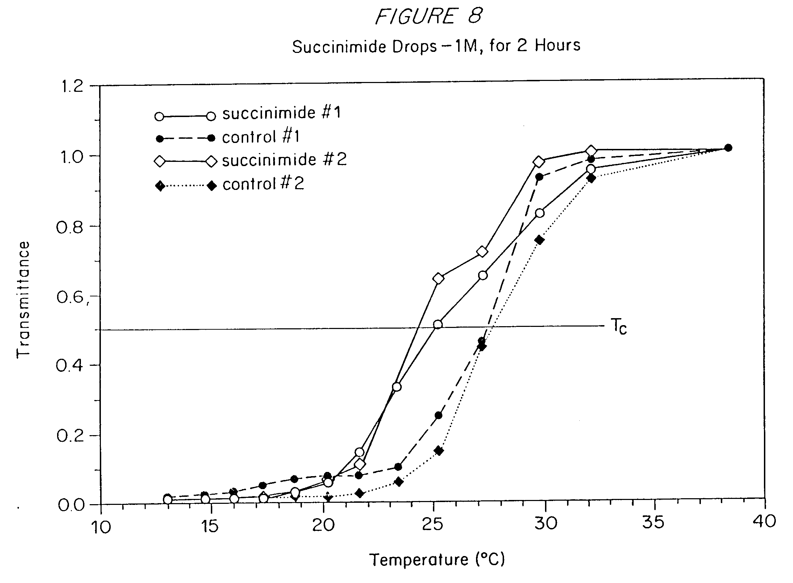

Data for succinimide in which the change in $T_c$ is plotted against reagent concentration is given in Figure 6(a). Data for ethosuximide in which the change in $T_c$ is plotted against reagent concentration is given in Figure 6(b).

Example 11: Reduction of Phase Separation Temperature in Whole Rat Eyes Using Succinimide

Freshly removed rat eyes were placed in solutions of physiological saline which contained 0, 100, 200 and 300 mM succinimide. The eyes were allowed to incubate for 24 hours at 4°C. The lenses were then removed and $T_c$ values were determined. The change in $T_c$ as it relates to the succinimide concentration was determined as:

$$\frac{dT_c}{d[C]} = 55.6 °C/Mole$$

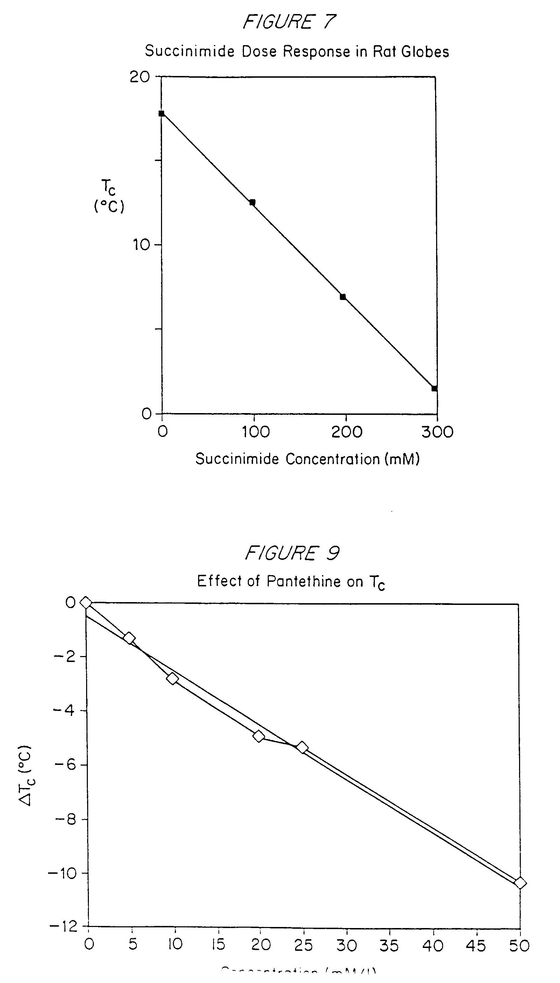

Data in which the change in $T_c$ is plotted against reagent concentration is given for this example in Figure 7.

Example 12: Reversal of Cataract Formed In Vivo by Sodium Selenite Via Succinimide

Sodium selenite was administered to 15 day old Sprague-Dawley rats to induce cataracts. After 4 or 5 days, the selenite-induced-cataracts which formed in the eyes were removed by removing the complete eye globes from each animal. Each globe was subsequently immersed in a 300 mM/l succinimide solution. In the presence of the succinimide, each cataract was found to disappear. Thus, the method of this example provided an actual reversal of in vivo cataracts.

10

Example 13: Reduction of Phase Separation Temperature in a Living Organism Using Succinimide

In two animals, a 1 molar solution of succinimide in phosphate buffered saline, PBS, was administered as eye drops to one eye and PBS alone was administered to the opposite eye as a control. After 2 hours, the lenses were removed and the $T_c$ in each lens was measured.

The $T_c$ in the lenses treated with succinimide was found to be about 3.5°C lower than the $T_c$ of lenses treated with the PBS control. These results demonstrated that topical administration of a phase separation inhibitor such as succinimide can effectively introduce the reagent to the interior of the lens and subsequently lower the phase separation temperature of lens proteins. A plot of lens transmittance versus lens temperature for both succinimide treated and control lenses is presented in Figure 8.

Example 14: In Vivo Administration of Pantethine

A single intraperitoneal injection of approximately 600 mg/Kg pantethine 15 minutes prior to X-irradiation prevented formation of cataracts produced by X-rays in Sprague-Dawley rats. These results can be seen in Figure 1, previously described.

Example 15: In Vitro Reduction of Phase Separation Temperature Using Pantethine

Concentrated bovine nuclear homogenate was assayed to be approximately 280 mg/ml. A solution of pantethine in phosphate buffer (0.1 M, pH 7), was prepared having concentrations of 0.50, 0.25, 0.20, 0.10 and 0.05 Molar. 10 microliter samples of the pantethine solutions were added to a second set of 90 microliter samples of concentrated bovine nuclear homogenate. A control sample was prepared by adding 10 microliters of buffer solution to 90 microliters of concentrated bovine nuclear homogenate.

The samples were allowed to stand for about 24 hours and the $T_c$ values for each were determined. The results indicated that pantethine is a strong phase separation inhibitor, lowering the phase separation temperature approximately 210°C/Mole. Data for pantethine, in which the change in $T_c$ is plotted against reagent concentration is given in Figure 9.

The results illustrated in the above examples indicate that natural and physiologically compatible reagents have the potential to protect against and reverse cataract formation by acting as phase separation inhibitors.

Although the reagents of the present invention have been described with reference to traditional methods of administration, such as drops into the eye, in tablet form or by injection, other means familiar to those skilled in the art of drug delivery could be utilized. For example, drug encapsulated in polymer matrices could be implanted into or adjacent the eye for sustained linear release over time. Either degradable (for example, polyanhydrides, polyorthoesters and polylactic acids) or non-degradable (for example, ethylene vinyl acetate and polystyrene) polymers could be used. Drug could also be injected directly into the aqueous humor.

Modifications and variations of the present invention, including methods and reagents for the prevention or reversal of cataract formation, will be obvious to those skilled in the art from the foregoing detailed description. Such modifications and variations are intended to come within the scope the appended claims.

**Claims**

1.  A reagent for the in vivo prevention or reversal of phase separation cataract formation in a human or animal patient comprising:
    a compound having two actions, the first action decreasing phase separation temperature of lens cytoplasmic proteins below the patient's body temperature and the second action inhibiting the formation of high molecular weight aggregates,
    wherein said compound is able to diffuse into the lens and is non-toxic to the lens and surrounding tissue, having no substantial effect on eye color and viscoelastic properties of the lens affecting visual acuity.

2.  The compound of claim 1 wherein said compound is selected from the group consisting of succinimide, N-hydroxy succinimide, ethosuximide and pantethenic acid derivatives.

3.  The compound of Claim 2 wherein said compound comprises pantethine.

4. The compound of claim 2 further comprising a physiological acceptable buffer.

5. The compound of claim 1 wherein said compound is selected from the group consisting of succinimide, N-hydroxy succinimide, ethosuximide, pantethenic acid derivatives, S-3-(amino-2-hydroxypropyl) phosphorothioate, S-2-(3-aminopropylaminoethyl) phosphorothioate and other phosphorothioates and is in a form suitable for topical administration to the patient's eye.

6. The compound of claim 1 wherein said compound is selected from the group consisting of pantethenic acid derivatives, S-3-(amino-2-hydroxypropyl) phosphorothioate, S-2-(3-aminopropylaminoethyl) phosphorothioate and other phosphorothioates and is in a form suitable for systemic administration.

7. The compound of claim 1 further comprising a biocompatible polymeric matrix for controlled delivery of said compound to the patient's eye.

8. A method for preventing or reversing cataract formation in vivo in a human or animal patient comprising:
   selecting a compound having two actions, the first action decreasing the phase separation temperature of lens cytoplasmic proteins below the patient's body temperature and the second action inhibiting the formation of high molecular weight aggregates, wherein said compound is able to diffuse into the lens and is non-toxic to the lens and surrounding tissue, having no substantial effect on eye color and viscoelastic properties of the lens affecting visual acuity; and
   administering the compound to the patient in a dose sufficient to prevent or reverse cataract formation.

9. The method of claim 8 wherein said compound is delivered prior to formation of high molecular weight aggregates.

10. The method of claim 8 wherein said compound is selected from the group consisting of succinimide, N-hydroxy succinimide, ethosuximide and pantethenic acid derivatives.

11. The method of Claim 10 wherein said compound comprises pantethine.

12. The method of claim 8 further comprising dispersing said compound in a physiologically acceptable buffer.

13. The method of claim 8 wherein said compound is administered by delivering said compound to the eye by topical administration.

14. The method of claim 13 wherein said compound is selected from the group consisting of succinimide, N-hydroxy succinimide, ethosuximide, pantethenic acid derivatives, S-3-(amino-2-hydroxypropyl) phosphorothioate, 2-2(3-aminopropylaminoethyl) phosphorothioate and other phosphorothioates and is administered to the patient prior to formation of high molecular weight aggregates.

15. The method of claim 8 further comprising encapsulating said compound in a form suitable for systemic delivery.

16. The method of claim 15 further comprising administering said compound orally.

17. The method of claim 15 wherein said compound is selected from the group consisting of pantethenic acid derivatives, S-3-(amino-2-hydroxypropyl) phosphorothioate, S-2-(3-aminopropylaminoethyl) phosphorothioate and other phosphorothioates and is administered to the patient systemically prior to formation of high molecular weight aggregates.

18. The method of claim 8 further comprising incorporating said compound into a biocompatible polymeric matrix for controlled delivery of said compound to the eye.

19. The method of claim 18 further comprising implanting said compound-polymeric matrix in a suitable location in the patient.

EP 0 538 911 A2

FIG.I

13

FIGURE 2

FIGURE 3

FIGURE 4

FIGURE 5

Effect of Natural Reagent on $T_C$

Each data point represents average of duplicate experiments.

15

FIGURE 6b

Ethosuximide Dose Response in
Concentrated Lens Homogenate

FIGURE 6a

Succinimide Dose Response in
Concentrated Lens Homogenates

## FIGURE 8

### Succinimide Drops — 1M, for 2 Hours

## FIGURE 7

### Succinimide Dose Response in Rat Globes

## FIGURE 9

### Effect of Pantethine on $T_C$